# EUROPÄISCHE PATENTSCHRIFT

(11) **EP 0 616 995 B1**
(45) Veröffentlichungstag und Bekanntmachung des Hinweises auf die Patenterteilung: **18.12.1996**
(21) Anmeldenummer: 94103289.8
(22) Anmeldetag: 04.03.1994
(51) Int. Cl.: C07C 45/72, C07C 45/56, C07C 49/76

(54) **Verfahren zur Herstellung von 1,3,5-Triacetylbenzol**
Process for the preparation of 1,3,5-triacetylbenzene
Procédé pour la préparation de 1,3,5-triacétylbenzène

(30) Priorität: 13.03.1993 DE 4308085
(43) Veröffentlichungstag der Anmeldung: 28.09.1994
(73) Patentinhaber: BASF Aktiengesellschaft, 67063 Ludwigshafen (DE)
(72) Erfinder: Rittinger, Stefan, Dr., D-67059 Ludwigshafen (DE); Rieber, Norbert, Dr., D-68259 Mannheim (DE)

(56) Entgegenhaltungen:
- US-A- 2 920 108
- CHEMICAL ABSTRACTS, vol. 108, no. 23, 6. Juni 1988, Columbus, Ohio, US; abstract no. 203986, MURAHASHI S ET AL 'Palladium-promoted transformation of.beta.-amino ketones to enaminones'

## Beschreibung

Die vorliegende Erfindung betrifft ein Verfahren zur Herstellung von 1,3,5-Triacetylbenzol durch Umsetzung von 1-Aminovinylmethylketonen mit Säuren.

Verfahren zur Herstellung von 1,3,5-Triacetylbenzol sind bekannt (s. z.B.: DE-A-912 209; DE-A-10 59 453; US-A-2 920 108; Bull. Acad. Sci. USSR, Sct. Chim., 883 (1953); Bull. Chem. Soc. Jpn. (1987) 60, Seiten 3285 - 3290). Die beschriebenen Einsatzstoffe sind jedoch sämtlich entweder nicht im technischen Maßstab verfügbar oder sehr hochpreisige Chemikalien.

Der vorliegenden Erfindung lag daher die Aufgabe zugrunde, den zuvor genannten Nachteilen abzuhelfen.

Demgemäß wurde ein neues und verbessertes Verfahren zur Herstellung von 1,3,5-Triacetylbenzol der Formel I 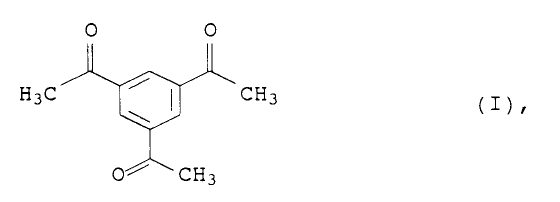 gefunden, welches dadurch gekennzeichnet ist, daß man 1-Aminovinylmethylketone der allgemeinen Formel II 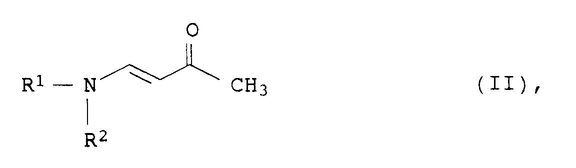 in der R¹ und R² C₁- bis C₂₀-Alkyl, C₃- bis C₈-Cycloalkyl, C₁- bis C₂₀-Hydroxyalkyl, Aryl, C₇- bis C₁₂-Phenalkyl und C₇- bis C₁₂-Alkylphenyl oder gemeinsam eine gegebenenfalls ein- bis vierfach durch C₁- bis C₄-Alkyl substituierte gegebenenfalls durch Sauerstoff, Stickstoff oder Schwefel unterbrochene C₂- bis C₇-Alkylenkette bedeuten, in Gegenwart von Säuren bei Temperaturen von 0 bis 150°C und Drücken von 0,01 bis 50 bar umsetzt und die 1-Aminovinylmethylketone II aus Diacetylen und sekundären Aminen erhält, wobei man
a) aus dem bei der Acetylenerzeugung resultierenden Spaltgas einen Teilstrom, der Diacetylen der Formel III

   H-C≡C-C≡C-H (III),

   enthält, durch Absorption abtrennt und
b) diesen Teilstrom mit sekundären Aminen der allgemeinen Formel IV 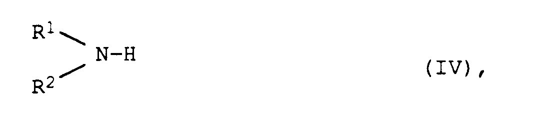 in der R¹ und R² die obengenannten Bedeutungen haben, und Wasser gegebenenfalls in einem Verdünnungsmittel bei Temperaturen von 0 bis 150°C und Drücken von 0,01 bis 5 bar umsetzt.

Das erfindungsgemäße Verfahren läßt sich wie folgt durchführen:

Die 1-Aminovinylmethylketone II können in Gegenwart von Säuren gegebenenfalls in einem inerten Lösungsmittel bei Temperaturen von 0 bis 150°C, bevorzugt 0 bis 100°C, besonders bevorzugt 20 bis 80°C und Drücken von 0,01 bis 50 bar, bevorzugt 0,1 bis 5 bar, besonders bevorzugt bei Atmosphärendruck (Normaldruck) sowohl diskontinuierlich als auch bevorzugt kontinuierlich in der Flüssigphase umgesetzt werden.

Als Säuren eignen sich beispielsweise Mineralsäuren wie Salzsäure, Schwefelsäure, Salpetersäure und Phosphorsäure, Sulfonsäuren wie Methansulfonsäure und p-Toluolsulfonsäure.

Das Molverhältnis von Säure zum 1-Aminovinylmethylketon II beträgt in der Regel 1 : 1 bis 50 : 1, bevorzugt 1 : 1 bis 10 : 1, besonders bevorzugt 1 : 1 bis 3 : 1.

Als inerte Lösungsmittel eignen sich Formamide, z.B. Dialkylformamide wie Dimethylformamid oder Lactame, bevorzugt N-Methylpyrrolidon, Alkohole, z.B. C₁- bis C₂₀-Alkanole wie Methanol und Ethanol, aromatische Kohlenwasserstoffgemische, z.B. C₇- bis C₂₀-Alkylaryle wie Benzol, Toluol, o-Xylol, m-Xylol, p-Xylol, Xylol-Isomerengemische, bevorzugt die üblicherweise bei der Spaltung von Kohlenwasserstoffen entstehenden Aromatengemische (Rückstandsöle) oder Glykolether wie Ethylenglykol-diethylether.

Das vorliegende Verfahren eignet sich im besonderen zur Verwertung von diacetylenhaltigen Teilströmen. Das - bei der Zerlegung von Spaltgasen aus der Spaltung von Kohlenwasserstoffen unter den Bedingungen der Acetylensynthese als verdünnter Teilstrom - anfallende Gemisch höherer Acetylene kann ohne weitere physikalische oder chemische Behandlung unmittelbar mit wäßrigen Lösungen von sekundären Aminen bei Temperaturen von 0 bis 150°C, bevorzugt 30 bis 130°C, besonders bevorzugt 40 bis 120°C und Drücken von 0,01 bis 5 bar, bevorzugt 0,1 bis 2,5 bar, besonders bevorzugt 0,5 bis 1,5 bar, in der Regel bei Normaldruck (Atmosphärendruck), umgesetzt werden.

Die technischen Varianten zur Erzeugung und Aufbereitung der erfindungsgemäß verwendeten Spaltgase ("Crack-Gase") ist z.B. aus Ullmann's Encyclopedia of Industrial Chemistry, V. Edition, A1, 1985, Seite 97 bis 145, insbesondere Seite 111, Fig. 13 bekannt, wobei Kohlenwasserstoffe (z.B. Erdgas oder auch höhersiedende Fraktionen) bei den für die Acetylenentstehung notwendigen, hohen Temperaturen gespalten und die resultierenden Produkte unmittelbar nach der Reaktionszone (Spaltzone) durch Eindüsen einer Flüssigkeit (z.B. Wasser oder Öl) rasch abgekühlt ("Quench") werden. Die Zusammensetzung der Spaltgase ist abhängig von den zur Spaltung eingesetzten Ausgangsstoffen, sowie den Spaltungsbedingungen.

Die erfindungsgemäß verwendeten Teilströme fallen bei der Zerlegung des Spaltgases durch eine Serie von physikalischen Trennvorgängen, bevorzugt durch Absorptions-/Desorptionsprozesse in einer Serie von Wasch- und Stripkreisläufen, in mehreren Teilströmen an. Die verwendbaren, höhere Acetylene enthaltenden Teilströme haben gegenüber dem Spaltgas deutlich höhere Konzentrationen an Diacetylen.

Eine weitere Aufbereitung des Einsatzstromes gemäß DE-A-21 57 537 ist nicht notwendig. Sowohl flüssige Einsatzströme als auch gasförmige, durch Strippen (Desorption) der Waschlösungen erhältliche Teilströme zeichnen sich gegenüber dem Spaltgas durch ein für die gewünschte Umsetzung zu Aminovinylmethylketon günstigeres Verteilungsprofil der höheren Acetylene aus. Es sind nur noch unwesentliche Mengen anderer, höherer Acetylene (Pentadiin, Hexadiin, etc.) enthalten, da diese vorher abgeschieden werden. Dies führt zu einer hohen Reinheit des erhaltenen Produktes.

Die Abtrennung der höheren Acetylene aus dem Spaltgas erfolgt in bevorzugter Weise so, daß die höheren Acetylene in einer Vorwäsche des das Gesamtacetylen enthaltenden Spaltgases mit einer Flüssigkeit absorbiert werden. Diese mit Diacetylen angereicherte Waschlösung kann dann noch in einem Vakuumstripper entgast werden, wobei die Gasfraktion nochmals vor oder unmittelbar nach einer eventuellen Kompression durch Zumischen eines Inertgasanteils stabilisiert wird.

Diacetylenhaltige Kohlenwasserstoffkondensate (sog. BTX-Fraktion) aus diesem Gasstrom eignen sich gleichermaßen vorteilhaft für die beschriebene Umsetzung von Diacetylen zu 1-Aminovinylmethylketon. Dies bewirkt für diese Vorgehensweise den zusätzlichen Vorteil, daß nach Abreaktion des Diacetylens destillativ die von Diacetylen befreite BTX-Fraktion erhalten werden kann, welche beispielsweise wieder als Einsatzstoff zur Crackung verwendet werden kann.

Als Absorptionsmittel in der Spaltgasaufbereitung eignen sich Wasser, hochsiedende Kohlenwasserstoffe wie Rückstandsöle, N-Alkyllactame mit C₁- bis C₃-Alkyl, bevorzugt N-Methylpyrrolidon, C₁- bis C₅-Alkohole, bevorzugt Methanol, Säureamide, bevorzugt Dimethylformamid, alkylierte cyclische Harnstoffe, bevorzugt Dimethylpropylenharnstoff, primäre, sekundäre und tertiäre C₁- bis C₆-Amine und Ammoniak.

Zur Verdünnung der aus dem Absorbens entfernten diacetylenhaltigen Gase eignen sich Inertgase wie Kohlenwasserstoffe, Kohlenmonoxid, Synthesegas, Armgas, Stickstoff oder Gemische dieser Gase, bevorzugt Erdgas und/oder Acetylen.

Das inertisierte Gasgemisch enthält in der Regel bevorzugt 55 bis 85 Vol-% Inertgas, 1 bis 30 Vol-% Diacetylen und 10 bis 20 Vol-% andere Bestandteile wie Acetylen, Vinylacetylen und Benzol. Der Anteil des Diacetylens im inertisierten Gasgemisch beträgt in der Regel 1 bis 30 Vol-%, bevorzugt 5 bis 20 Vol-%. Die Obergrenze wird durch die Grenze des explosiven Selbstzerfalls im Inertgas bestimmt.

Typische gasförmige, höhere Acetylene enthaltende Teilströme haben folgende Zusammensetzung:
- 58 Vol-% Methan, 18 Vol-% Diacetylen, 5 Vol-% Stickstoff, 4 Vol-% Acetylen, 4,5 Vol-% Vinylacetylen, 4 Vol-% Benzol, 2 Vol-% Ethan, 2 Vol-% Cyclopentadien, 2,5 Vol-% Restbestandteile.

Typische flüssige, höhere Acetylene enthaltende Teilströme haben folgende Zusammensetzung:
- 32 Vol-% Benzol, 28 Vol-% Toluol, 17 Vol-% Xylol (BTX), 8 Vol-% Styrol, 6 Vol-% Diacetylen,
- Methanol (89 Vol-%), Acetylen (0,6 Vol-%), Diacetylen (2,2 Vol-%), Vinylacetylen (0,4 Vol-%), Cyclopentadien (1 Vol-%), Benzol (1,8 Vol-%) und Toluol (1,3 Vol-%).

Die Umsetzung des Teilstroms mit wäßrigen sekundären Aminen kann sowohl diskontinuierlich als auch kontinuierlich gestaltet werden. Bei Nutzung eines gasförmigen Teilstroms sind Verfahren, die eine gute Gasverteilung in Flüssigkeiten erzielen, z.B. die Verwendung von Apparateteilen wie Begasungsring, Lochboden, verdichtende Begasungseinrichtungen, Sprühreaktor oder Absorberturm vorteilhaft.

Nicht vollständig umgesetztes Amin kann destillativ vom Produkt oder dem bei unmittelbarer Weiterverarbeitung erhältlichen Folgeprodukt abgetrennt und in die Synthesestufe a) zurückgeführt werden.

Das Gasgemisch aus der Spaltgasaufbereitung hat gewöhnlich gegenüber der Umgebung leicht erhöhte Betriebstemperatur. Die Vorabscheidung von leicht kondensierbaren Gasbestandteilen in Abscheidebehältern in der Reaktorzuleitung bei Umgebungstemperatur verbessert die Reinheit des Rohproduktes.

Die Substituenten R¹ und R² in den Verbindungen II und IV haben folgende Bedeutungen:

R¹ und R²
- unabhängig voneinander
- C₁- bis C₂₀-Alkyl, bevorzugt C₁- bis C₈-Alkyl, besonders bevorzugt C₁- bis C₄-Alkyl wie Methyl, Ethyl, n-Propyl, iso-Propyl, n-Butyl, iso-Butyl, sec.-Butyl und tert.-Butyl,
- C₃- bis C₈-Cycloalkyl, bevorzugt C₅- bis C₈-Cycloalkyl wie Cyclopentyl, Cyclohexyl, Cycloheptyl und Cyclooctyl, besonders bevorzugt Cyclopentyl und Cyclohexyl,
- C₁- bis C₂₀-Hydroxyalkyl, bevorzugt C₂- bis C₈-Hydroxyalkyl, besonders bevorzugt C₂- bis C₄-Hydroxyalkyl wie 2-Hydroxyethyl und 3-Hydroxypropyl,
- Aryl wie Phenyl, 1-Naphthyl und 2-Naphthyl, bevorzugt Phenyl, 1-Naphthyl und 2-Naphthyl, besonders bevorzugt Phenyl,
- C₇- bis C₁₂-Alkylphenyl wie 2-Methylphenyl, 3-Methylphenyl, 4-Methylphenyl, 2,4-Dimethylphenyl, 2,5-Dimethylphenyl, 2,6-Dimethylphenyl, 3,5-Dimethylphenyl und 2,3,4-Trimethylphenyl,
- C₇- bis C₁₂-Phenylalkyl wie Benzyl, 1-Phenethyl, 2-Phenethyl, 1-Phenyl-propyl, 2-Phenyl-propyl, 1-Phenyl-butyl, 2-Phenyl-butyl, 3-Phenyl-butyl, bevorzugt Benzyl, 1-Phenethyl und 2-Phenethyl, oder
- gemeinsam
- eine gegebenenfalls durch ein bis vier C₁- bis C₄-Alkylreste substituierte gegebenenfalls durch Sauerstoff, Stickstoff oder Schwefel unterbrochene C₂- bis C₇-Alkylenkette wie -(CH₂)₂-, -(CH₂)₃-, -(CH₂)₄-, -(CH₂)₅-, -(CH₂)₆-, -(CH₂)₇-, -(CHCH₃)-, -(CH₂)₂-O-(CH₂)₂-, -(CH₂)₂-N-(CH₂)₂- und -(CH₂)₂-S-(CH₂)₂-.

1,3,5-Triacetylbenzol eignet sich als Zwischenprodukt (CA-A-1 223 880, Rec. Trav. Chim. 77, 782 bis 791 (1958); US-A-2 920 108) sowie für die Herstellung von Comonomeren für die Styrolpolymerisation (DE-A-962 118; US-A-3 153 102).

In dem folgenden Beispiel ist das inertisierte, diacetylenhaltige Gasgemisch mit HA-Gas bezeichnet. Das Diacetylen im HA-Gas bzw. im Abgas nach der Reaktion wurde gaschromatographisch auf einer gepackten Säule (20 % Reoplex 400 auf Chromosorb PAW) mit Trägergas N₂ (35 ml/min.) und FID-Detektion bestimmt. Die Konzentrationen sind in Vol-% angegeben.

### Beispiel

In einem 500-ml-Rührbehälter wurden eine Mischung aus 67 g Morpholin und 33 g H₂O vorgelegt und auf 80°C erhitzt. 15 l/h eines Teilstroms des HA-Abgases der Acetylenproduktion wurden durch ein Einleitungsrohr mit Glasfritte während 12 Betriebsstunden durch die Reaktionslösung hindurchgeleitet. Die Diacetylenkonzentration des HA-Gases am Reaktoreingang schwankte von 4 bis 9 %. Bei einer durchschnittlichen Abreicherung des Diacetylens von über 90 % resultierte 130 g dunkelge-färbte Reaktionslösung mit einem Restmorpholingehalt von 18 % und einem Gehalt von 60 % 1-Morpholinovinylmethylketon. Nach Aufsetzen einer Destillationsbrücke wurden bei ca. 200 mbar/75°C ein Gemisch aus Wasser und Morpholin abdestilliert. Restliches Amin wurde durch Verminderung des Drucks auf 20 mbar entfernt. Der schwarzbraun gefärbte Rückstand wurde über 6 h 1000 ml einer auf 40°C erwärmten 0,5 N HCl-Lösung zugetropft. Die nunmehr aufgeklarte, hellgelbe Lösung wurde bei 40°C im Vakuum auf ca. die Hälfte des Volumens eingeengt, wobei Triacetylbenzol begann auszukristallieren. Die Fällung wurde durch Abkühlung auf 0°C verstärkt, worauf durch Filtration 80 g (78,5 %) Triacetylbenzol in Form farbloser Kristalle (Sdp. 156 bis 158°C) isoliert wurden. Die Mutterlauge enthielt nur noch Spuren an Produkt (0,6 g n. GC-Flächen-%-Analyse).

## Patentansprüche

1. Verfahren zur Herstellung von 1,3,5-Triacetylbenzol der Formel I 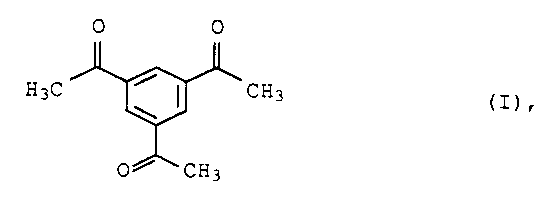 dadurch gekennzeichnet, daß man 1-Aminovinylmethylketone der allgemeinen Formel II 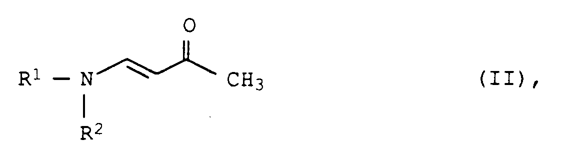 in der R¹ und R² C₁- bis C₂₀-Alkyl, C₃- bis C₈-Cycloalkyl, C₁- bis C₂₀-Hydroxyalkyl, Aryl, C₇- bis C₁₂-Phenalkyl und C₇- bis C₁₂-Alkylphenyl oder gemeinsam eine gegebenenfalls ein- bis vierfach durch C₁- bis C₄-Alkyl substituierte gegebenenfalls durch Sauerstoff, Stickstoff oder Schwefel unterbrochene C₂- bis C₇-Alkylenkette
bedeuten, in Gegenwart von Säuren bei Temperaturen von 0 bis 150°C und Drücken von 0,01 bis 50 bar umsetzt und die 1-Aminovinylmethylketone II aus Diacetylen und sekundären Aminen erhält, indem man
a) aus dem bei der Acetylenerzeugung resultierenden Spaltgas einen Teilstrom, der Diacetylen der Formel III
H-C≡C-C≡C-H (III),
enthält, durch Absorption abtrennt und
b) diesen Teilstrom mit sekundären Aminen der allgemeinen Formel IV 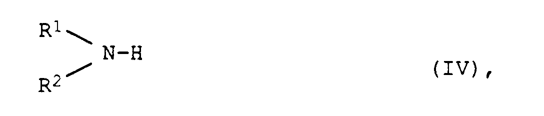 in der R¹ und R² die obengenannten Bedeutungen haben, und Wasser gegebenenfalls in einem Verdünnungsmittel bei Temperaturen von 0 bis 150°C und Drücken von 0,01 bis 5 bar umsetzt.

## Claims

1. A process for preparing 1,3,5-triacetylbenzene of the formula I 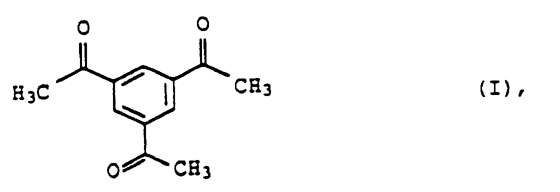 which comprises reacting 1-aminovinyl methyl ketones of the general formula II 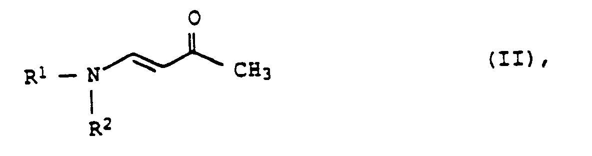 where R¹ and R² are C₁-C₂₀-alkyl, C₃-C₈-cycloalkyl, C₁-C₂₀-hydroxyalkyl, aryl, C₇-C₁₂-phenalkyl and C₇-C₁₂-alkylphenyl or together are a C₂-C₇-alkylene chain which is unsubstituted or substituted once to 4 times by C₁-C₄-alkyl and may be interrupted by oxygen, nitrogen or sulfur,
in the presence of acids at from 0 to 150°C under from 0.01 to 50 bar, and obtaining the 1-aminovinyl methyl ketones II from diacetylene and secondary amines by
a) removing by absorption a part-stream which contains diacetylene of the formula III
H-C≡C-C≡C-H (III),
from the cracked gas resulting from acetylene production, and
b) reacting this part-stream with secondary amines of the general formula IV 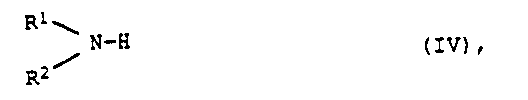 where R¹ and R² have the abovementioned meanings, and water in the presence or absence of a diluent at from 0 to 150°C under from 0.01 to 5 bar.

## Revendications

1. Procédé de préparation du 1,3,5-triacétylbenzène de la formule I 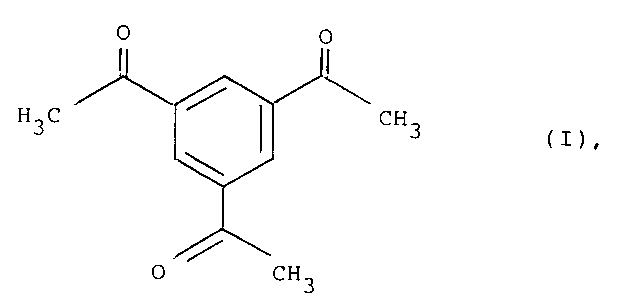 caractérisé en ce que l'on fait réagir des 1-aminovinylméthylcétones de la formule générale II 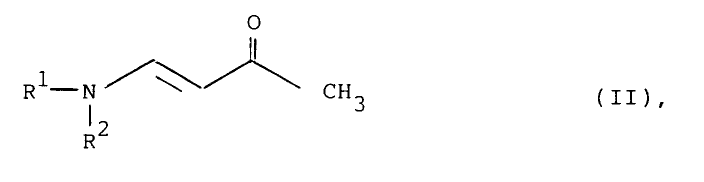 dans laquelle R¹ et R² représentent chacun un radical alkyle en C₁ à C₂₀, cycloalkyle en C₃ à C₈, hydroxyalkyle en C₁ à C₂₀, aryle, phénalkyle en C₇ à C₁₂ et alkylphényle en C₇ à C₁₂, ou représentent en commun une chaîne alkylène en C₂ à C₇ éventuellement une à quatre fois substituée par des radicaux alkyle en C₁ à C₄ et éventuellement interrompue par de l'oxygène, de l'azote ou du soufre,
en présence d'acides, à des températures de 0 à 150°C et sous des pressions de 0,01 à 50 bars et on obtient les 1-aminovinylméthylcétones II à partir de diacétylène et d'amines secondaires, pour autant que
a) à partir du gaz de fission résultant de l'obtention de l'acétylène, on sépare par absorption un courant partiel qui contient du diacétylène de la formule III
H-C≡C-C≡C-H (III),
et
b) on fasse réagir ce courant partiel avec des amines secondaires de la formule IV 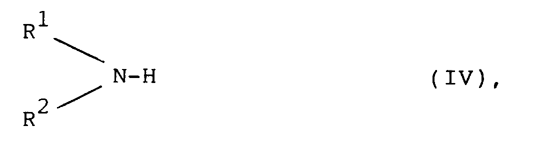 dans laquelle R¹ et R² possèdent les significations qui leur ont été attribuées ci-dessus et de l'eau, éventuellement dans un diluant, à des températures de 0 à 150°C et sous des pressions de 0,01 à 5 bars.
